# EUROPÄISCHE PATENTANMELDUNG

(11) **EP 2 263 731 A2**
(43) Veröffentlichungstag der Anmeldung: **22.12.2010**
(21) Anmeldenummer: 10184929.7
(22) Anmeldetag: 16.02.2001
(51) Int. Cl.: A61M 16/08, A61M 16/10, F16L 59/02, F16L 53/00, A61M 16/16

(54) **Atemgasschlauchanordnung zur Zufuhr eines Atemgases**

(30) Priorität: 18.02.2000 DE 10007506
(62) Teilanmeldung aus: 08003371.5
(71) Anmelder: MAP Medizin-Technologie GmbH, 82152 Martinsried (DE)
(72) Erfinder: Huber, Petra, 85435 Erding (DE); Lang, Bernd, 82166 Gräfelfing (DE); Biener, Achim, 85445 Aufkirchen (DE); Heidmann, Dieter, Castle Hill, NSW 2154 (AU)
(74) Vertreter: Vossius & Partner

(57) **Zusammenfassung**

Die Erfindung betrifft eine Atemgasschlauchanordnung zur Zufuhr eines Atemgases zu einer Atemmaske mit einer flexiblen Schlauchleitung 16, gekennzeichnet durch einen flexiblen Mantelkörper 1, der die flexible Schlauchleitung 16 umgibt und sich entlang der Schlauchleitung 16 erstreckt.

## Beschreibung

Die Erfindung betrifft eine Atemgasschlauchanordnung zur Zufuhr eines Atemgases zu einer Person.

Derartige Atemgasschlauchanordnungen finden insbesondere im Bereich der Schlafmedizin zur Behandlung schlafbezogener Atmungsstörungen Anwendung. So kann beispielsweise bereits durch einen vergleichsweise geringen Atemgasüberdruck eine pneumatische Schienung der oberen Atemwege erreicht werden, wodurch auf wirkungsvolie Weise der Gefahr von Atemwegsobstruktionen vorgebeugt werden kann.

Die Behandlung schlafbezogener Atmungsstörungen durch eine kontinuierliche Überdruokbeatmung wird aligemein als CPAP-Therapie bezeichnet. Bei den hierzu verwendeten bekannten CPAF-Geräten ist üblicherweise in einem schailisolierten Gehäuse eine Fördereinrichtung, insbesondere ein Gebläse angeordnet, dessen Förderdruck, ggf. über eine elektronische Regeleinrichtung auf die Atmungstätigkeit des Patienten abgestimmt wird. Das durch die Fördereinrichtung geförderte Atemgas, in der Regel Umgebungsluft, wird über eine flexible Atemgasschlauchanordnung dem Patienten zugeführt. Hierzu werden im Regelfall Atemmasken verwendet, die auf die Nase des Patienten aufgesetzt werden, ohne hierbei den Mund des Patienten abzudecken. Es hat sich gezeigt, daß die CPAP-Therapie eine effektive und physiologisch gut verträgliche Therapiemethode zur Behandlung schlafbezogener Atmungsstörungen darstellt. Die zur Verbindung der patientenseitig angeordneten Atemmaske mit dem CPAP-Gerät vorgesehene flexible Schlauchleitung wird jedoch oft als störend empfunden.

Der Erfindung liegt die Aufgabe zugrunde, eine Vorrichtung zur Zufuhr eines Atemgases zu einem Patienten zu schaffen, die sich durch einen erhöhten Anwendungskomfort auszeichnet.

Diese Aufgabe wird erfindungsgemäß durch die im Patentanspruch 1 angegebene Atemgasschlachanordnung gelöst.

Dadurch wird es auf vorteilhafte Weise möglich, einen unmittelbaren Berührungskontakt der Schlauchleitung mit dem Patienten zu vermeiden und die Schlauchleitung in einer unter ästhetischen Gesichtspunkten vorteilhaften Weise zu verbergen. In besonders vorteilhafter Weise wird auch der Kondensatbildung in der Schlauchleitung vorgebeugt, so daß auch im Falle hoher absoluter Feuchtigkeit des Atemgases sich im Inneren der flexiblen Schlauchleitung keine Kondensattropfen bilden können.

Gem. einer besonders bevorzugten Ausführungsform der Erfindung ist der flexible Mantelkörper aus einem welchen Textilmaterial - insbesondere Fleecematerial - gefertigt.

Der flexible Mantelkörper ist in vorteilhafter Weise derart dimensioniert, daß die flexible Schlauchleitung darin zwanglos aufgenommen ist. Vorzugsweise beträgt der innendurchmesser des flexiblen Mantelkörpers wenigstens das 1,3-Fache des Außendurchmessers der flexiblen Schlauchleitung. Durch das zwischen der Außenwandung der flexiblen Schlauchleitung und dem flexiblen Mantelkörper gebildete Luftpolster wird eine weitere Verbesserung des Anwendungskomforts sowie der Isolierwirkung erreicht.

Es ist möglich, den flexiblen Mantelkörper aus einem mehrlagigen Material zu bildern, das erst im Außenbereich eine unter ästhetischen Gesichtspunkten ausgewählte, angenehme Außenlage aufweist.

Der flexible Mantelkörper weist gem. einer besonders bevorzugten Ausführungsform der Erfindung im Bereich seiner Enden eine Schließeinrichtung auf, durch welche der Endbereich des flexiblen Mantelkörpers verengt, beispielsweise eingeschnürt, und vergleichsweise festsitzend an der flexiblen Schlauchleitung fixiert werden kann. As geeignete Fixiereinrichtung wird gem. einer besonders bevorzugten Ausführungsform der Erfindung eine Zurrschnur verwendet, die durch Ösen und/oder einen Hohlsaum hindurchgeführt ist und ein Zusammenschnüren des entsprechenden Endes des flexiblen Mantels ermöglicht. Alternativ hierzu oder auch in Kombination damit ist es möglich, im Endbereich des flexiblen Mantels eine Klettverschlußeinrichtung vorzusehen, über weiche das Ende des flexiblen Mantelkörpers entsprechend verengt werden kann.

Die Wanddicke des flexiblen Mantelkörpers liegt vorzugsweise im Bereich von 1 - 7 mm. Das verwendete Material kann beispielsweise ein Vollmaterial (Vliesmaterial) oder auch ein mehrlagiges Material sein. In besonders vorteilhafter Weise ist eine flexible Textillage auf eine Polsterlage, beispielsweise aus Schaumstoffmaterial aufkaschiert.

Der flexible Mantelkörper ist vorzugsweise als schlauchartiger Körper ausgebildet und hierbei auf die flexible Schlauchleitung aufgeschoben.

Alternativ dazu ist es auch möglich, den flexiblen Mantelkörper aus einem Streifenmaterial zu bilden, das eine sich in dessen Längsrichtung erstreckende Verbindungseinrichtung aufweist. Diese Verbindungseinrichtung ist in vorteilhafter Weise durch ein Klettverschlußband gebildet. Alternativ hierzu sind auch Reißverschiuß-Verbindungseinrichtungen oder auch Knopflochanordnungen sowie Druckknöpfe möglich.

In besonders vorteilhafter Weise ist es möglich, im Bereich des CPAP-Gerätes sowie vorzugsweise auch im Bereich der Atemmaske Verbindungsmittel vorzusehen, über welche der flexible Mantelkörper zuverlässig fixiert werden kann. In vorteilhafter Weise bestehen diese Fixiermittel aus einem Vlies- oder Kletthakenband, das im jeweiligen Endbereich (Maske oder CPAP-Gerät) am Schlauch fixiert vorzugsweise angeklebt oder geklemmt ist.

Es ist gemäß einer besonders bevorzugten Ausführungsform der Erfindung auch möglich, im Bereich der Enden des Mantelkörpers elastische Bündchen vorzusehen durch welche das Mantelmaterial des Mantelkörpers auf den Schlauch oder die Schlauch-Endstücke gedrängt wird. Hierzu ist es beispielsweise möglich, ein Gummiband im Endbereich des Mantelkörpers vorzusehen. Das Gummiband ist hierbei vorzugsweise in einem Hohlsaum aufgenommen.

Es ist in vorteilhafte Weise möglich, im Bereich der Auflagezonen des Mantelkörpers auf dem Schlauch eine rutschfeste beispielsweise gummierte Lage vorzusehen.

Die Wärme- und Schallisolationswirkung kann in vorteilhafer Weise durch die Materialbeschaffenheit und den Aufbau des Mantelkörpers bedarfsgerecht abgestimmt werden. Eine besonders hohe Wärmeisolationswirkung wird erreicht, indem mehrere Lagen Fleece-Material verarbeitet werden. Es ist möglich, die Lagen des Fleece-Materiales vorzugsweise in Verbindung mit Deck- und Futterlagen abzusteppen oder zu quilten.

Zumindest der Außenbereich des Mantelkörpers ist vorzugsweise mehrfarbig ausgebindet. Diese Mehrfarbigkeit wird vorzugsweise durch Vernähen mehrfarbiger Textilabschnitte erreicht, wobei die Verbindungsnähte hierbei gleichzeitig als Quiltnähte dienen.

Es ist möglich, in den Mantelkörper eine Band- oder Stab-artige Versteifungseinlage einzubinden, so daß bei einer schlauchartigen Ausgestaltung des Mantelkörpers eine Aufzieh-Hilfe erreicht wird.

Im Falle einer längsgeteilten Ausführungsform des Mantelkörpers ist es möglich, alternativ zu Klett- oder Knopf- insbesondere Druckknopf-Verbindungen hier einen Längsreißverschluß vorzusehen. Dieser Längsreißverschluß ist vorzugsweise derart überlappt ausgebildet, daß die Reißverschiußstruktur nach außen überdeckt ist. Es ist auch möglich, hier eine Magnetverschlußeinrichtung vorzusehen.

Gemäß einer besonders bevorzugten Ausführungsform der Erfindung ist in den Mantelkörper eine Heizeinrichtung integriert. Diese Heizeinrichtung kann durch eine elektrische Widerstandsheizung gebildet sein. Gemäß einer besonders bevorzugten Ausführungsform umfaßt die Heizeinrichtung eine Niedervoltheizfolie, wobei das Leistungsaufnahmeverhalten dieser Heizfolie derart gewählt ist, daß die Heizfolie eine vorgegebenen Heiztemperatur selbstregelnd nicht überschreitet. Die Heizfolie kann in den Mantelkörper eingenäht sein, wobei vorzugsweise das Wärmeisolationsvermögen des Mantelkörpers außerhalb der Heizfolie größer ist als im Bereich zwischen Heizfolie und Schlauch. Alternativ hierzu ist es auch möglich, den Schlauch als beheizbaren Atemgasschlauch auszubilden.

Die Spannungsversorgung der Heizeinrichtung erfolgt vorzugsweise über eine Niedervoltleitung die mit einem Anschlußstecker versehen ist, welcher in eine entsprechende Spannungsversorgungsbuchse eines Netzgerätes - oder vorzugsweise eine, an einem CPAP-Gerät, oder einem Luftbefeuchter vorgesehene Spannungsversorgungsdose einsteckbar ist. Durch einen derart beheizbar ausgebildeten Mantelkörper wird neben einer zuverlässigen Vermeidung von Kondensationserscheinungen auch Temperierung des Atemgases ermöglicht.

Es ist möglich, im Bereich des Mantelkörpers Isolationsmittel zur Wärmestrahlungsabschirmung vorzusehen. Vorzugsweise ist hierbei auf einer an die Schlauchleitung angrenzenden Innenseite des Mantelkörpers eine metallisierte beispielsweise Aluminium, Silber- oder Gold-bedampfte Superisolationsfolie vorgesehen.

Gemäß einer besonders bevorzugten Ausführungsform der Erfindung sind an dem Mantelkörper wenigstens eine - vorzugsweise mehrere Fixierstellen vorgesehen, über welche der Mantelkörper - und damit die gesamte Schlauchanordnung aufgehängt oder definiert in ihrer räumlichen Bewegbarkeit festgelegt werden können. Diese Fixierstellen können beispielsweise durch Ösen ausgebildet sein. Vorzugsweise sind zwei bis vier derartige Ösen zueinander beabstandet entlang der Schlauchleitung angeordnet.

Gemäß einer besonders bevorzugten Ausführungsform der Erfindung ist der Mantelkörper derart ausgestaltet, daß dieser einen Behälterabschnit bildet, in welchen der übrige Teil des Mantelkörpers hineingesteckt werden kann. Dieser in den Mantelkörper integrierte Behälterabschnitt kann als flacher Beutel oder Taschenbereich ausgebildet sein der durch Aufnähen einer flexiblen Lage auf eine Außenfläche des Mantelkörpers gebildet ist. Es ist auch möglich, den überwiegenden Teil des Mantelkörpers in den Innenbereich eines seiner Längsendabschnitte hinein zu zwängen. Hierzu ist vorzugsweise ein Längsabschnitt des Mantelkörpers sackartig ausgebildet.

Eine besonders gewichtssparende Ausführungsform des Mantefkörpers wird dadurch erreicht, daß wenigstens eine der isolierenden Lagen als Luftpolster-Lage ausgebildet ist.

Der Mantelkörper ist gemäß einem besonderen Aspekt der vorliegenden Erfindung derart ausgebildet, daß dessen Biegeverhalten in Längsrichtung des Mentelkörpers betrachtet, variiert. So ist es beispielsweise möglich, im maskennahen Bereich des Mantelkörpers eine hohe Flexibilität vorzusehen, wogegen im gerätenahen Bereich der Mantelkörper steifer ausgebildet ist. Das Verformungsverhalten des Mantelkörpers kann hierbei durch Gelenk- und/oder Biegematerialeinlagen bestimmt werden.

Der Mantelkörper ist gemäß einer besonders bevorzugten Ausführungsform der Erfindung mit einem Knickschutz beispielsweise in Form von Spiral- oder schlangenlinienartig verlaufenden Einlagen versehen. Hierdurch wird es möglich, den in dem Mantelkörper aufgenommenen Atemgasschlauch dünnwandig - ggf. als Einweg-Leicht-Schlauch auszubilden.

Der Mantelkörper kann gemäß einer besonders bevorzugten Ausführungsform der Erfindung auch länger ausgebildet sein als die eigentliche Schlauchleitung. Hierdurch wird es möglich, durch den Mantelkörper auch Zusatzfunktionselemente abzudecken. So ist es in Verbindung mit dem Mantelkörper möglich, beispielsweise modular an die Schlauchleitung angekoppelte Elemente durch den Mantelkörper abzudecken und zu einer nach außen hin abgeschlossen erscheinenden Einheit zu kombinieren.

Es ist insbesondere möglich, einen rohr- oder schlauchförmig ausgebildeten Luftbefeuchter, ein Gasanalysemodul, einen Gasfiuß-Sensor, eine Druckschleuse oder dgl. maskennah modulartig an die Schlauchleitung anzukoppeln und durch den Mantelkörper abzudecken und bedarfsweise zu isoiieren. Durch den Mantelkörper sind hierbei in vorteilhafter Weise etwaige Zusatzleitungen elektrischer, optischer, oder auch pneumatischer Art abgedeckt.

Der Mantelkörper bildet hierbei ein flexibles vorzugsweise wärme- und schallisolierendes, die einzelnen Module übergreifendes, und hierbei zusammenfassendes, Gehäuseteil.

Der Mantelkörper kann mit mehreren Öffnungen versehen sein, durch welche Leitungseinrichtungen beispielsweise einer Wasserfalle aus dem Mantelkörper herausgeführt werden können. Diese Öffnungen sind vorzugsweise über eine Deckeleinrichtung verschließbar.

Die folgenden Punkte sind bevorzugte Ausführungsformen der vorliegenden Erfindung:
1. Atemgasschlauchanordnung zur Zufuhr eines Atemgases zu einer Atemmaske mit einer flexiblen Schlauchleitung (16), gekennzeichnet durch einen flexibien Mantelkörper (1), der die flexible Schlauchleitung (16) umgibt und sich entlang der Schlauchleitung (16) erstreckt.
2. Atemgasschlauchanordnung nach Punkt 1, **dadurch gekennzeichnet, daß** der flexible Mantelkörper (1) aus einem wärmeisolierenden Material gebildet ist.
3. Atemgasschlauchanordnung nach Punkt 1 oder 2, **dadurch gekennzeichnet, daß** der flexible Mantelkörper (1) aus einem Fleecematerial gebildet ist.
4. Atemgasschlauchanordnung nach wenigstens einem der Punkte 1 bis 3, **dadurch gekennzeichnet, daß** der flexible Mantelkörper (1) mehrere Msterialschichten (8, 9, 10) aufweist.
5. Atemgasschlauchanordnung nach wenigstens einem der Punkte 1 bis 4, **dadurch gekennzeichnet, daß** der flexible Mantelkörper (1) im Bereich seiner Stirnenden mit einer Verschlußeinrichtung (2, 4; 3, 5) versehen ist.
6. Atemgasschlauchanordnung nach wenigstens einem der Punkte 1 bis 5, **dadurch gekennzeichnet, daß** die Verschlußeinrichtung durch eine Zurr- oder Klettverschlußeinrichtung gebildet ist.
7. Atemgasschlauchanordnung nach wenigstens einem der Punkte 1 bis 6, **dadurch gekennzeichnet, daß** der flexible Mantelkörper (1) in Längsrichtung geteilt ist.
8. Atemgasschlauchanordnung nach wenigstens einem der Punkte 1 bis 7, **dadurch gekennzeichnet, daß** der flexible Mantelkörper (1) eine sich entlang einer Seitenkante erstreckende Klettverschlußverbindungseinrichtung (17) aufweist.
9. Vorrichtung zur Zufuhr eines Atemgases zu einem Patienten mit einer Fördereinrichtung zur Förderung des Atemgase, einer Atemmaske und einer flexiblen Schlauchleitung zur Koppelung der Atemmaske mit der Fördereinrichtung, gekennzeichnet durch einen flexiblen Mantelkörper (1), der die flexible Schlauchleitung umgibt und sich von der Aternmaske zur Fördereinrichtung hin erstreckt.
10. Mantelkörper für eine Atemgasleitung mit einer flexiblen beispielsweise aus einem Textilmaterial gebildeten Wandung, gekennzeichnet durch eine in die Wandung integrierte Heizeinrichtung.
11. Mantelkörper nach Punkt 10, **dadurch gekennzeichnet, daß** die Heizeinrichtung eine sich um die Schlauchleitung erstreckende Heizfolle umfaßt.
12. Mantelkörper für eine Atemgasleitung bestehend aus einem wärmeisolierenden flexiblen Textilmaterial, mit einer Fixiereinrichtung zur Fixierung wenigstens eines Mantelkörperendabschnittes an der Atemgasleitung.
13. Flexible Atemgasschlauchanordnung, insbesondere nach einem der vorangehenden. Punkte , zur Zufuhr eines Atemgases zu einer Person, aufweisend:
   eine flexible Schlauchleitung;
   einen flexiblen Mantelkörper;
   eine Heizeinrichtung zum Erwärmen des durch die Schlauchleitung (123) geförderten Gases;
   an den Enden der Schlauchleitung (123) vorgesehene Anschlusselemente, um die Schlauchleitung (123) mit einer Atemmaske (112) bzw. einer Gebläseeinrichtung zu verbinden; wobei
   das Anschiusselement zur Verbindung mit einer Gebläseeinrichtung ais Anschlussstecker ausgebildet ist, der einen Atemgasdurchgangsquerschnitt (130) sowie Kontaktelemente (128) aufweist, über die die Heizeinrichtung mit Spannung versorgt wird.
14. Anschlusselement zum Verbinden einer Gebläseeinrichtung mit einer Atemgasschlauchanordnung, insbesondere nach einem der vorangehenden Ansprüche, zur Zufuhr eines Atemgases zu einer Person, wobei die Atemgasschlauchanordnung eine Heizeinrichtung aufweist, und wobei das Anschlusselement an einem Ende einer Schlauchleitung (123) vorgesehen ist, um die Schlauchleitung mit einer Gebläseeinrichtung zu verbinden, und wobei das Anschlusselement als Anschlussstecker ausgebildet ist, der einen Atemgasdurchgangsquerschnitt sowie Kontaktelemente bereitstellt, über die die Heizeinrichtung mit Spannung versorgt wird.
15. CPAP-Vorrichtung, aufweisend:
   eine Fördereinrichtung zur Förderung von Atemgas über eine flexible Atemgasschlauchanordnung, insbesondere nach einem der vorangehenden Ansprüche, zu einem Patienten;
   eine elektronisch Regeleinrichtung zum Abstimmen des Förderdruckes der Fördereinrichtung; und
   ein Anschlussstruktur, zum Verbinden mit einem Anschlusselement gemäß Punkt 13, wobei die Anschlussstruktur eine Spannungsversorgung aufweist, um elektrische Spannung an die Kontaktelemente des Anschlusssteckers bereitzustellen, um Spannung an die Heizeinrichtung bereitzustellen, um das durch die Atemgasschlauchanordnung (123) geförderte Gas zu erwärmen. abzudecken und zu einer nach außen hin abgeschlossen erscheinenden Einheit zu kombinieren.

Es ist insbesondere möglich, einen rohr- oder schlauchförmig ausgebildeten Luftbefeuchter, ein Gasanalysemodul, einen Gasftuß-Sensor, eine Druckschleuse oder dgl, maskennah modulartig an die Schlauchleitung anzukoppeln und durch den Mantelkörper abzudecken und bedarfsweise zu isolieren. Durch den Mantelkörper sind hierbei in vorteilhafter Weise etwaige Zusatzleitungen elektrischer, optischer, oder auch pneumatischer Art abgedeckt.

Der Mantelkörper bildet hierbei ein flexibles vorzugsweise wärme- und schallisolierendes, die einzelnen Module übergreifendes, und hierbei zusammenfsssendes, Gehäuseteil.

Der Mantelkörper kann mit mehreren Öffnungen versehen sein, durch weiche Leitungseinrichtungen beispielsweise einer Wasserfalle aus dem Mantelkörper herausgeführt werden können. Diese Öffnungen sind vorzugsweise über eine Deckeieinrichtung verschließbar.

Weitere Einzelheiten der Erfindung ergeben sich aus der nachfolgenden Beschreibung unter Bezugnahme auf die beigefügte Zeichnung. Es zeigen:
- Fig. 1: eine perspektivische Ansicht einer CPAP-Atemgasschlauchanordnung, mit dem erfindungsgemäß vorgeschlagenen, aus einem textilen Welchmaterial gefertigten, Mantelkörper;
- Fig. 2a: eine vereinfachte Schnittansicht, durch einen Abschnitt eines mehrlagig ausgebildeten Mantelkörpers;
- Fig. 2b: eine vereinfachte Schnittansicht, durch einen Abschnitt eines einlagig ausgebildeten Mantelkörpers;
- Fig. 2c: eine vereinfachte Schnittansicht, durch einen Abschnitt eines zweilagig mit einer textilen Decklage ausgebildeten Mantelkörpers;
- Fig. 3: eine perspektivische Darstellung zur Erläuterung einer im Endbereich des flexiblen Mantelkörpers vorgesehenen Fixiereinrichtung;
- Fig. 4: eine Ausführungsform des flexiblen Mantelkörpers als längsgeteilter Körper einschließlich der sich entlang der entsprechenden Längskante erstreckenden, hier als Klettverschluß ausgebildeten Verbindungseinrichtung;

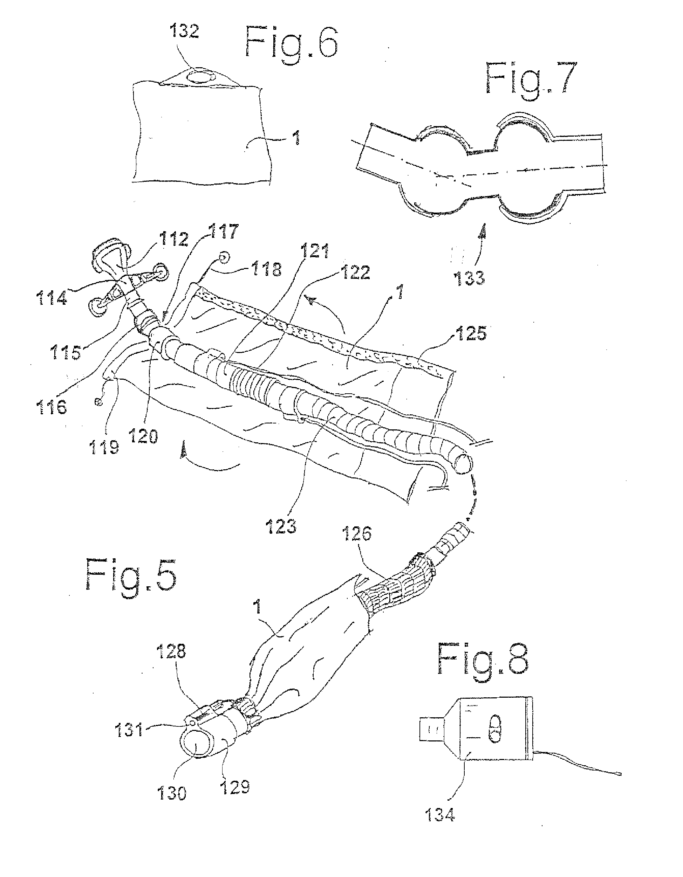

- Fig.5: eine perspektivische Skizze zur Erläuterung einer weiteren Ausführungsform einer Atemgasschlauchanordnung;
- Fig.6: eine Skizze eines Abschnittes des Mantelkörpers hier mit Ösenabschnitten zur Aufhängung des Mantelkörpere;
- Fig.7: eine Skizze zur Erläuterung eines in den Mantelkörper abschnittsweise einsetzbaren Gelenkeinsatzes;
- Fig.8: eine Skizze einer kartuschenartig ausgebildeten Gebläseeinrichtung (CPAP-Druckquelle) zum Einsatz in den Mantelkörper.

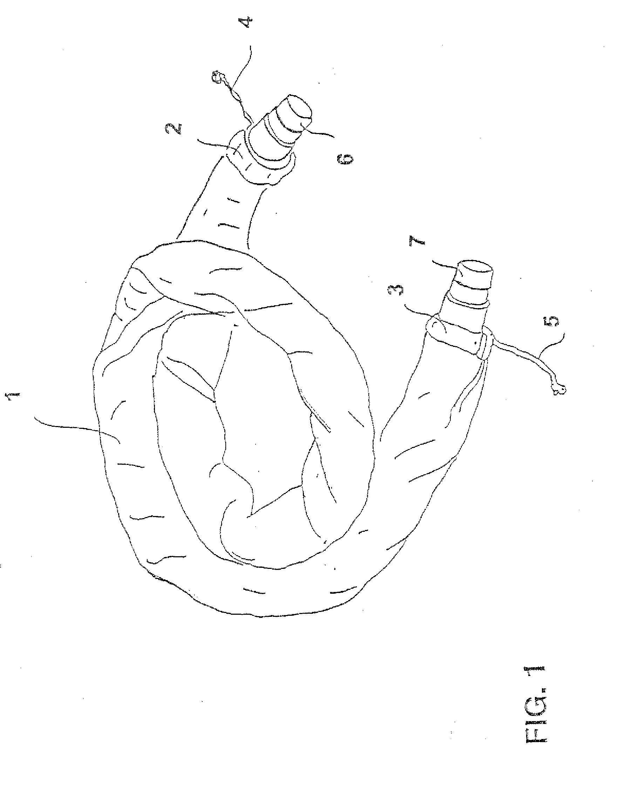

Die in Fig. 1 dargestellte Atemgasschlauchanordnung umfaßt eine hier nicht sichtbare, aus einem spiralverstärkten Kunststoffmaterial gebildete flexible Schlauchleitung und einen aus einem isolierenden Textilmaterial - hier Vliesmaterial - gebildeten isolierenden Mantelkörper 1. Der Mantelkörper 1 ist aus einem schmalen Stoffstreifen gebildet, der entlang einer Längsnaht (Overiocknaht) zu einem Schlauch vernäht ist. Im Endbereich des Mantelkörpers 1 ist bei der hier dargestellten Ausführungsform jeweils ein Hohlsaum 2, 3 ausgebildet, durch welchen jeweils eine Schnur 4, 5 hindurchgeführt ist. Durch entsprechendes Ziehen der Schnüre 4, 5 können die Endbereichs des Mantelkörpers 1 zusammengebogen werden, wodurch auf vorteilhafte Weise der Mantelkörper 1 an der flexiblen Schlauchleitung fixiert ist. Zum Anschluß der flexiblen Schlauchleitung an ein CPAP-Gerät, bzw. an eine Atemmaske sind im jeweiligen Schlauchendbereich Elasto-merbuchsen 6, 7 vorgesehen, die elastisch auf eine entsprechend komplementäre Anschlußstruktur auf, bzw. in diese eingesteckt werden können.

Der Mantelkörper 1 ist derart dimensioniert, daß die flexible Schlauchleitung darin im wesentlichen zwanglos aufgenommen ist. Das hierbei zwischen der Innenfläche des Mantelkörper 1 und der Außenfläche der flexiblen Schlauchleitung gebildete Luftpolster führt zu einer ohne Gewichtszunahme verbesserten Wärmeisolierung und verleiht der Atemgasschlauchanordnung zudem einen angenehmen Polstereffekt.

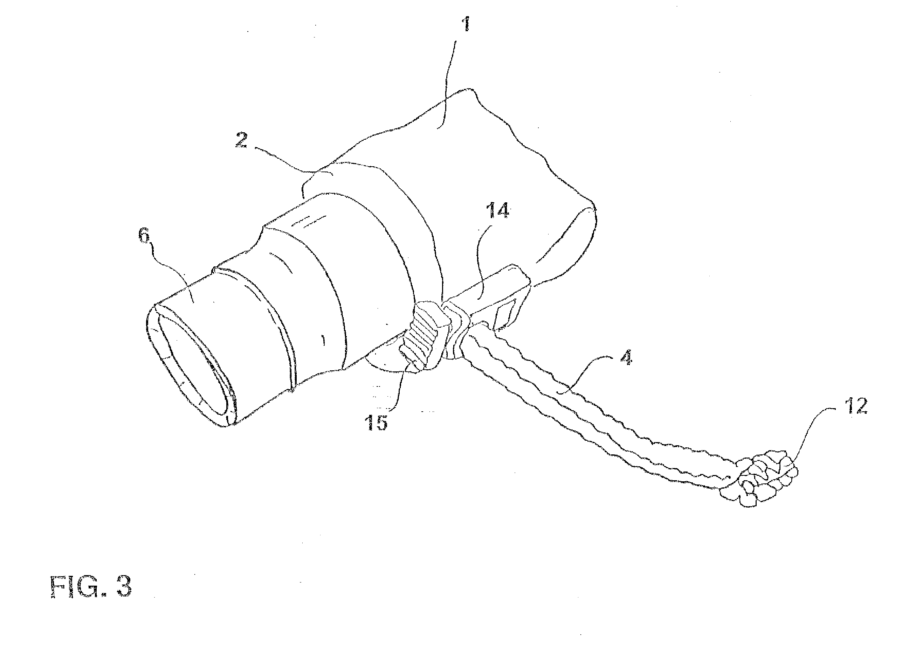

Zur Fixierung der Schnüre 4, 5 in einer entsprechenden Zurrposition sind - wie nachfolgend unter Bezugnahme auf Fig. 3 noch ausführlich erläutert werden wird, vorzugsweise Fixiermittel (hier nicht sichtbar) vorgesehen.

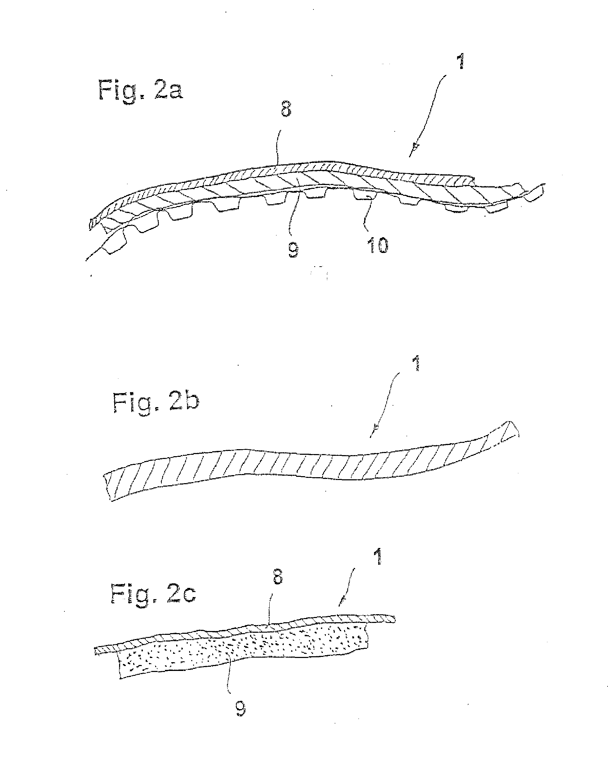

Der Mantelkörper 1 ist vorzugsweise, wie in Fig. 2a andeutungsweise dargestellt, mehrlagig ausgebildet. So kann beispielsweise als oberste, bzw. äußere Decklage eine unter ästhetischen Gesichtspunkten ausgewählte textile Decklage 8 auf einen polsternden und isolierenden Unterbau, der hier aus einer Schaumstofflage 9 und einer Noppenfolie 10 besteht, aufkaschiert werden. Die Verbindung zwischen den einzelnen Lagen kann durch entsprechende Verklebung, durch Nähte oder vorzugsweise durch Wärmekaschieren erfolgen. So ist es beispielsweise möglich, die isolierende Schaumstofflage 9 auf einem Schaumstoffmaterial zu bilden, dessen Deckfläche bei einer vorbestimmten Schmelztemperatur mit der äußeren Lage 8 und der Noppenfolie 10 verschweißt.

Alternativ zu dem beschriebenen mehrlagigen Aufbau ist es auch möglich, den Mantelkörper 1 aus einem Vollmaterial, vorzugsweise aus einem Vlies- oder Filzmaterial zu bilden, wie dies in Fig. 2b dargestellt ist. Vorzugsweise werden hierbei Materialien verwendet, die eine einfache Reinigung, vorzugsweise einen Kochwaschvorgang gestatten.

In Fig. 2c ist eine weitere Ausführungsform des Wandungsmaterials des flexiblen Mantelkörpers 1 dargestellt, das hier aus einer polsternden und wärmeisolierenden Schaumstofflage 9 und einer aus einem dekorativen Gewebematerial gebildeten Decklage 8 besteht. Die Verbindung zwischen der Decklage 8 und dem Schaumstoffmaterial 9 ist hier durch einen Flammkaschiervorgang erreicht.

In Fig. 3 ist eine bevorzugte Ausführungsform einer Fixiereinrichtung zur Fixierung des flexiblen Mantelkörpers 1 im Endbereich des flexiblen Atemgasschlauches dargestellt. Die Fixiereinrichtung umfaßt hier eine durch einen Hohlsaum 2 hindurchgeführte Schnur 4, die im Bereich ihrer herausgeführten Enden mit einem Knoten 12 versehen ist. Durch entsprechendes Ziehen an dem herausgeführten Abschnitt der Schnur 4, bzw. dem Knoten 12 kann das Ende des flexiblen Mantelkörpers 1 zusammengeschnürt werden.

Mittels einer Fixiereinrichtung 14 kann die Schnur 4 in der gespannten Stellung gehalten werden. Durch Betätigung einer hier als Druckknopf 15 ausgebildeten Löseeinrichtung kann die Schnur 4 gelockert und hierbei das zunächst eingeschnürte Ende des Mantelkörpers 1 geweitet werden. Eine besonders sichere Fixierung des Mantelkörpers 1 an der flexiblen Schlauchleitung kann dadurch erreicht werden, daß im Bereich des hier als elastomere Buchse 6 ausgebildeten Abschlußelementes eine Struktur ausgebildet ist, die mit dem entsprechenden Endabschnitt des Mantelkörper 1 in Eingriff bringbar ist. Beispielsweise kann die elastomere Buchse 6 mit einer Umfangsnut oder einem Umfangswulst versehen sein, an welchem der entsprechend zusammengeschnürte textile Mantelkörper 1 fixierbar ist.

Alternativ zu der hier dargestellten Zurranordnung ist es auch möglich, das Ende des Mantelkörpers 1, beispielsweise über eine Klettverschlußeinrichtung oder Schnallen an der flexiblen Schlauchleitung (nicht dargestellt) zu fixieren.

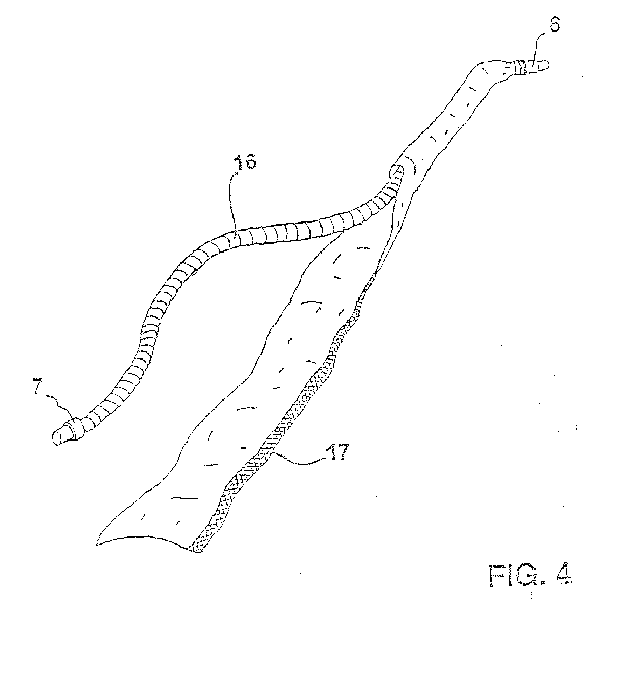

In Fig. 4 ist eine weitere Ausführungsform der Atemgasschlauchanordnung dargestellt, die hier eine flexible Schlauchleitung 16 aufweist, an deren Endbereich die elastomeren Buchsen 6, 7 vorgesehen sind. Abweichend von der vorangehend in Verbindung mit der in Fig. 1 beschriebenen Ausführungsform ist hier der flexible Mantelkörper 1 aus einem flexiblen Textilstreifen gebildet, der um die flexible Schlauchleitung 16 herumgeführt und entlang seiner Längskante mit einer Verbindungseinrichtung 17 versehen ist, so daß der um die Schlauchleitung 16 herumgeführte Materialstreifen ebenfalls schlauchartig die flexible Schlauchleitung

## Patentansprüche

1. Vorrichtung zur Zufuhr eines Atemgases zu einem Patienten mit einer Fördereinrichtung zur Förderung des Atemgases, einer Atemmaske und einer flexiblen Schlauchleitung zur Koppelung der Atemmaske mit der Fördereinrichtung, **gekennzeichnet durch** einen flexiblen Mantelkörper (1), der lösbar mit der Schlauchleitung verbunden ist und der die flexible Schlauchleitung umgibt und sich von der Atemmaske zur Fördereinrichtung hin erstreckt, wobei die Fördereinrichtung ausgebildet ist, in den Mantelkörper eingesetzt zu werden.

2. Vorrichtung nach Anspruch 1, wobei die Vorrichtung eine Heizeinrichtung aufweist, wobei vorzugsweise der Mantelkörper und/oder die Schlauchleitung eine Heizeinrichtung aufweisen

3. Vorrichtung nach Anspruch 1 oder 2, wobei an den Enden der Schlauchleitung (123) Anschlusselemente vorgesehen sind, um die Schlauchleitung (123) mit einer Atemmaske (112) bzw. einer Gebläseeinrichtung zu verbinden; wobei das Anschlusselement zur Verbindung mit der Gebläseeinrichtung als Anschlussstecker ausgebildet ist, der einen Atemgasdurchgangsquerschnitt (130) sowie Kontaktelemente (128) aufweist, über die die Heizeinrichtung mit Spannung versorgt wird.

4. Vorrichtung nach einem der vorstehenden Ansprüche, wobei der flexible Mantelkörper (1) aus einem Fleecematerial gebildet ist.

5. Vorrichtung nach einem der vorstehenden Ansprüche, wobei der flexible Mantelkörper (1) mehrere Materialschichten (8, 9, 10) aufweist.

6. Vorrichtung nach einem der vorstehenden Ansprüche, wobei der flexible Mantelkörper (1) im Bereich seiner Stirnenden mit einer Verschlusseinrichtung (2, 4; 3, 5) versehen ist.

7. Vorrichtung nach einem der vorstehenden Ansprüche, wobei die Verschlusseinrichtung durch eine Zurr- oder Klettverschlusseinrichtung gebildet ist.

8. Vorrichtung nach einem der vorstehenden Ansprüche, wobei der flexible Mantelkörper (1) in Längsrichtung geteilt ist.

9. Vorrichtung nach einem der vorstehenden Ansprüche, wobei der flexible Mantelkörper (1) eine sich entlang einer Seitenkante erstreckende Klettverschlussverbindungseinrichtung (17) aufweist.

10. Vorrichtung nach einem der vorstehenden Ansprüche, wobei der Mantelkörper oder die Atemgasleitung mit eine flexible, beispielsweise aus einem Textilmaterial gebildete, Wandung aufweist, **gekennzeichnet durch** eine in die Wandung integrierte Heizeinrichtung.

11. Vorrichtung nach einem der vorstehenden Ansprüche, wobei der flexible Mantelkörper (1) aus einem wärmeisolierenden Material gebildet ist.

12. Vorrichtung nach einem der vorstehenden Ansprüche, wobei die Heizeinrichtung eine sich um die Schlauchleitung erstreckende Heizfolie umfasst.

13. Vorrichtung nach einem der vorstehenden Ansprüche, wobei der Mantelkörper eine Fixiereinrichtung zur Fixierung wenigstens eines Mantelkörperendabschnittes an der Atemgasleitung aufweist.

14. Vorrichtung nach einem der vorstehenden Ansprüche, wobei die Vorrichtung eine CPAP-Vorrichtung ist.
